# EUROPEAN PATENT SPECIFICATION

(11) **EP 2 576 584 B1**
(45) Date of publication and mention of the grant of the patent: **07.01.2015**
(21) Application number: 11727731.9
(22) Date of filing: 01.06.2011
(51) Int. Cl.: C07J 31/00

(54) **METHODS AND COMPOUNDS FOR THE PREPARATION OF MONOFLUOROMETHYLATED BIOLOGICALLY ACTIVE ORGANIC COMPOUNDS**
VERFAHREN UND VERBINDUNGEN ZUR HERSTELLUNG MONOFLUORMETHYLIERTER BIOLOGISCH AKTIVER ORGANISCHER VERBINDUNGEN
PROCÉDÉS ET COMPOSÉS POUR PRÉPARER DES COMPOSÉS ORGANIQUES MONOFLUOROMÉTHYLÉS BIOLOGIQUEMENT ACTIFS

(30) Priority: 01.06.2010 PT 513810
(43) Date of publication of application: 10.04.2013
(73) Proprietor: Hovione Inter Limited, 6000 Lucerne 7 (CH)
(72) Inventor: LEITAO, Emilia, Perpétua, Tavares, P-2735-673 Sao Marcos (PT); HEGGIE, William, P-2450-656 Palmela (PT)
(74) Representative: Turner, Craig Robert
(86) International application number: PCT/GB2011/000835
(87) International publication number: WO 2011/151625

(56) References cited:
- EP-A1- 0 437 689
- WO-A1-02/12266
- WO-A1-94/14834
- WO-A1-2012/160338
- US-A- 3 399 179
- T. PATRICK ET AL: "Replacement of the carboxylic acid function with fluorine", CANADIAN JOURNAL OF CHEMISTRY, vol. 64, 1986, pages 138-141, XP002660069, NRC Research Press ISSN: 0008-4042 cited in the application

## Description

The carbon-fluorine bond is commonly found in pharmaceutical and agrochemical products, because it is generally metabolically stable and the fluorine atom acts as a bioisostere of the hydrogen atom (Ann M. Thayer "Fabulous Fluorine" Chemical and Engineering News, June 5, 2006, Volume 84, pp. 15-24). Nowadays around 20% of all pharmaceutical compounds and 30-40% of agrochemicals on the market contain fluorine. Fluorination and fluoroalkylation are the two major synthetic methods to prepare selectively fluorinated organic compounds. The monofluoromethylation (selective introduction of a CH₂F group into the organic molecule) is less studied than fluorination.

The exploration of di- and monofluoromethylated compounds as organic biologically active compounds has emerged recently. As a result, a variety of structurally diverse CH₂F- containing drugs have been developed, such as: Afloqualone, Fluticasone Propionate (Jinbo Hu; Wei Zhang; Fei wang; Chem. Commum., 2009, 7465-7478), the anaesthetic Sevoflurane and Fluticasone Furoate.

The efficient and selective incorporation of monofluoromethylated moieties into the organic molecule is beneficial for the synthesis of the target molecule. The process is usually carried out directly using CH₂FBr or indirectly, using CH₂BrI or CH₂ClI, among others. These compounds are known as hydrochlorofluorocarbons or freons (HCFCs), which is a subclass of chlorofluorocarbons (CFCs).

Every permutation of fluorine, chlorine, and hydrogen on the methane and ethane core has been examined and most have been commercialized. Furthermore, many examples containing bromine are known for higher numbers of carbon as well as related compounds. The use of this class of compounds include refrigerants, blowing agents, propellants in medicinal applications, and degreasing solvents (M. Rossberg et al. "Chlorinated Hydrocarbons" in Ullmann's Encyclopedia of Industrial Chemistry 2006, Wiley-VCH, Weinheim).

Unfortunately, due to their high stability, CFCs do not decompose in the lower atmosphere as many industrial chemicals do. In fact they are accumulating and eventually rise to the stratosphere. Ultraviolet radiation in the stratosphere breaks the CFCs apart, and the released chlorine atoms destroy the ozone layer. For this reason, the manufacture of such compounds is being phased out according to the Montreal Protocol (Pool, R. 1989. The elusive replacements for CFCs. Science 242: 666). Under the Montreal Protocol, it was agreed to start reducing their consumption and production in 2015.

The literature describes a method for replacing a carboxylic group with a fluorine group in a halogenated aliphatic carboxylic compound having the general formula, R-COOH, to prepare a fluorinated product having the general formula, R-F. The fluorodecarboxylation is carried out in the presence of XeF₂ (Timothy B. Patrick, Kamalesh K. Johri, David H. White, William S. Bertrand, Rodziah Mokhtar, Michael R. Kilboum, Michael J. Welch CAN. J. CHEM. Vol. 64,1986) or BrF₃ (Patent US4996371)

WO-94/14834 discloses the introduction of an -S-CH₂F group by the direct fluorination of an -S-CH₃ group using XeF₂. WO-02/12266 discloses the production of fluticasone furoate by the reaction of the corresponding 17-COSH intermediate with BrCH₂F.

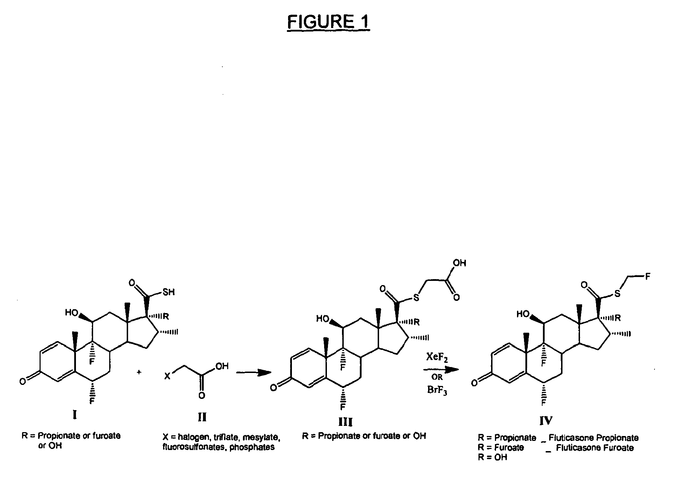

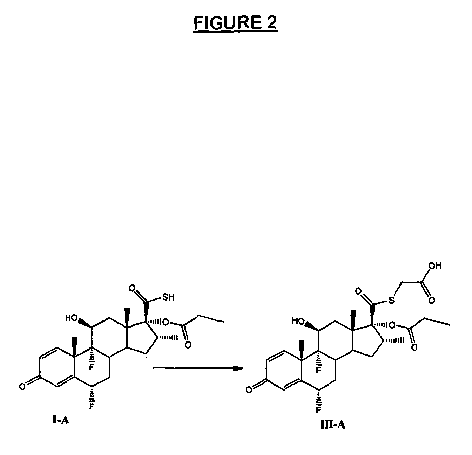

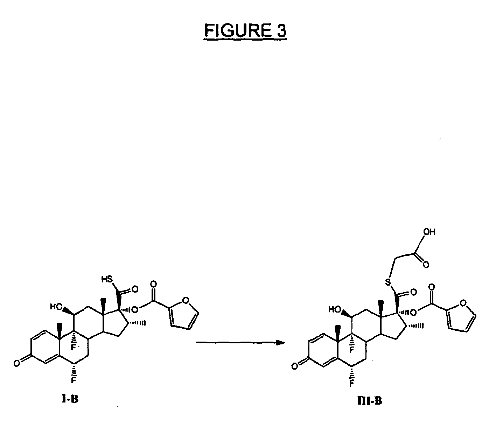

We have now discovered that, surprisingly, these reagents can be used as part of the synthesis of highly complex compounds, and can for example be applied in the synthesis of organic biologically active compounds, for example steroids, such as Fluticasone Propionate and Fluticasone Furoate as presented in Fig. 1. This avoids the use of bromofluoromethane or any other related substance that depletes the ozone layer. Fig. 1 illustrates the reaction of steroid (I) with X-acetic acid (II) to afford intermediate (III). Intermediate (III) is then fluorodecarboxylated to obtain Fluticasone Propionate or Fluticasone Furoate (IV).

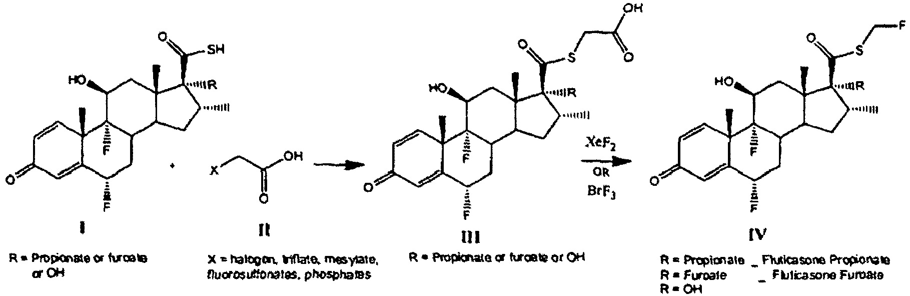

Any of the methods described above (amongst others) can be used for the preparation of the organic biologically active compounds which incorporate a "CH₂F" moiety.

According to a broad aspect of the present invention, there is provided a method of preparing an organic biologically active compound containing a "CH₂F" moiety, which method comprises the steps of: reacting a compound of formula R*-SH with X-acetic acid to yield an intermediate of formula R*-S-CH₂COOH; fluorodecarboxylating the intermediate of formula R*-S-CH₂COOH with a fluorodecarboxylating reagent to yield a compound of formula R*-S-CH₂F, wherein:
R*SH is an organic multifunctional molecule;
and X is halogen, triflate, mesylate, a fluorosulfonate or a phosphate.

By "organic multifunctional molecule" it will be understood we mean to refer to any organic molecule of general formula R*SH which can serve as a precursor to the organic biologically active compound of interest and which can react with X-acetic acid according to the above scheme. Typically, the organic multifunctional molecule will be a complex molecule, and the molecule will contain at least one functional group in addition to an -SH group. Molecules having a steroidal structure (eg steroid precursors for biologically active steroid compounds) are particularly preferred. The molecule may have more than one additional functional group in addtion to the -SH group.

Preferably, the molecule R*SH comprises one or more of the following functional groups: ketone, halogen, unsaturated hydrocarbon containing one or more carbon-carbon double bonds (ie an - ene group, for example, alkene), or hydroxyl. All four functional groups may be present if desired. The halogen is preferably fluorine.

Preferably, the compound of formula R*SH is a steroid molecule.

In a preferred aspect, the invention provides a method of preparing an organic biologically active compound containing a "CH2F" moiety, comprising the steps of: reacting a steroid of formula I with X-acetic acid of formula II to yield an intermediate of formula III; fluorodecarboxylating the intermediate of formula III with a fluorodecarboxylating reagent as defined herein to yield compound of formula IV, 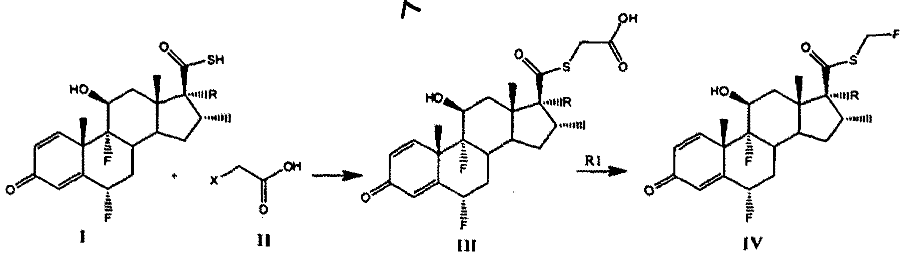 wherein:
R is propionate, furoate or hydroxyl and X is halogen, triflate, mesylate, a fluorosulfonate or a phosphate; and
R1 is a fluorodecarboxylating reagent as defined herein.

The fluorodecarboxylating reagent used in the invention is chosen from a group consisting of XeF₂ and BrF₃

For X-acetic acid, X is preferably halogen, and preferably the halogen is bromine.

In the compounds of formula I, III, and IV, R is preferably furoate or propionate.

The present invention also provides a compound of formula III, 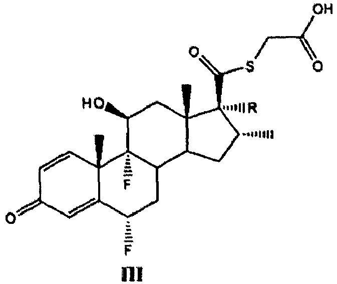 wherein R is propionate, furoate or hydroxyl.

The invention also provides and a process for preparing an organic biologically active compound using a compound of formula III wherein the organic biologically active compound contains a "CH₂F" moiety and using XeF₂ or BrF₃ as Huorodecarboxylating agent. Preferably, the organic biologically active compound containing a "CH₂F" moiety is a compound of formula IV, 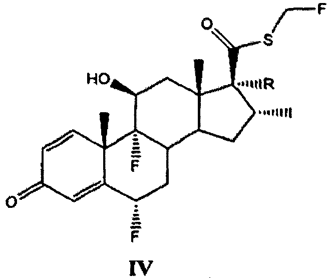 wherein R is furoate or propionate or hydroxyl.

For each of the steps in the method of the invention, the amount of reagent required (ie X-acetic acid or fluorodecarboxylating agent) per mole of substrate is suitably from about 0.9 to 7 mole equivalents. A range of about 1 to 2 mole equivalents is preferred, and is particularly suitable for the preparation of fluticasone and derivatives thereof.

Intermediate (III) can be prepared by the reaction of steroid (I) with an X-acetic acid (II) in an organic solvent and in the presence of an organic or inorganic base at a temperatures range within -70 °C and 70 °C. The product can be isolated and purified by precipitation in water or water with acid or water with base, by extraction with organic solvent and/or concentration, by recrystallization in organic solvent, and/or by column chromatography. Resin and activated charcoal can also be used during the work-up to purify the products.

The product of formula IV is prepared by fluorodecarboxylation of compound III using as fluordecarboxylating reagent XeF₂ and BrF₃ and can be isolated and purified by precipitation in water or water with acid or water with base, by extraction with organic solvent and/or concentration, by recrystallization in organic solvent, and/or by column chromatography. Resin and activated charcoal can also be used during the work-up to purify the monofluoromethylated products.

### Examples

The following examples are merely illustrative and not intended to limit the scope of the invention.

### Example 1

### Preparation of compound of formula III-A (S-acetic acid-6a,9 α -difluoro-11 β-hydroxy, 16 α -methyl-3-oxo-17 α -(propionyloxy)androsta-1,4-diene-17β-carbothiate), wherein the R is propionate

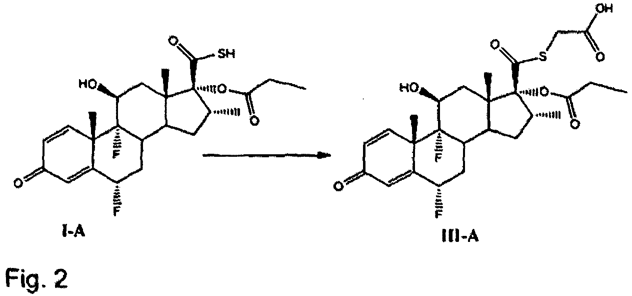

A solution of compound of formula I-A (1 g, 2.1 mmol), triethylamine (0.440 mL, 3.15 mmol), bromoacetic acid (0.330 g, 2.31 mmol) in dichloromethane (10 mL) was stirred at room temperature overnight. Water was added (10 mL) and the mixture extracted with dichloromethane (3x10 mL), dried with anhydrous MgSO₄, and concentrated to afford compound of formula III-A (1.451 g) as solid, as characterised further below:
1H NMR (CDCl₃), 400 MHz: δ 7.22 (1H, d, J=10.1 Hz), 6.42 (1H, s), 6.37 (1H, dd, J=10.1, J=1.6 Hz), 5.39 (1H, ddd, J=48.9, J=10.3, J=6.4 Hz), 4.38 (1H, d, J=9.08 Hz), 3.75 (1 H, d, J=16.0 Hz), 3.65 (1H, d, J=16.0 Hz), 3.38-3.34 (1 H, m), 3.12 (2H, dd, J=14.5 Hz, J=7.2 Hz), 2.41-2.21 (5H, m), 2.02-1.98 (1H, m), 1.90-1.72 (2H, m), 1.53 (3H, s), 1.11 (3H, t, J=7.4 Hz), 1.11 (3H, s), 0.98 (3H, d, J=7.04 Hz).
13C NMR (CDCl₃), 100 MHz: δ 196.1, 185.8, 172.9, 172.6, 161.9, 161.8, 151.3, 129.9, 121.0, 120.9, 100.0, 98.2, 96.3, 86.5 (JCF=183 Hz), 71.7, 71.3, 60.4, 48.9, 48.2, 48.0, 45.7, 43.0, 36.2, 35.6, 34.1, 33.8, 33.6, 32.9, 32.8, 32.7, 32.6, 27.7, 23.0, 17.2, 16.1, 14.1, 9.1, 8.5.
FT-IR values are as follows:
   FT-IR (KBr): 3407, 1743, 1670, 1631, 1608 cm⁻¹.

### Example 2

### Preparation of compound of formula III-B (S-acetic acid-6α,9 α -difluoro-17 α -[(2-furanyicarbonyl)oxy]-11ß-hydroxy-16 α-methyl-3-oxo-androsta-1,4-diene-17 β-carbothiate), wherein the R is furoate

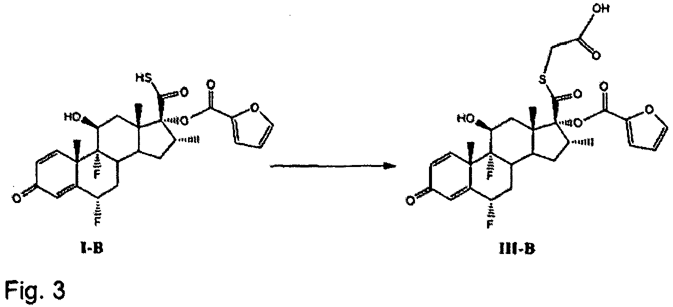

A solution of compound of formula I-B (1 g, 1.97 mmol), triethylamine (0.410 mL, 2.96 mmol), bromoacetic acid (0.302 g, 2.17 mmol) in dichloromethane (10 mL) was stirred at room temperature overnight. Water was added (10 mL) and the mixture extracted with dichloromethane (3x10 mL), dried with anhydrous MgSO₄, and concentrated to afford compound of formula III-B (1.429 g) as solid, as characterised further below:
1H NMR (CDCl₃), 400 MHz: δ 7.56 (1H, s), 7.21 (1H, d, J=10.1 Hz), 7.09 (1H, d, J=3.4 Hz), 6.49-6.48 (1H, m), 6.42 (1H, s), 6.37 (1H, dd, J=10.1, J=1.1 Hz), 5.39 (1H, ddd, J=48.8, J=10.8, J=6.4 Hz), 4.36 (1H, d, J=9.08 Hz), 3.75 (1H, d, J=15.8 Hz), 3.67 (1H, d, J=15.8 Hz), 3.46-3.42 (1H, m), 2.48-2.26 (4H, m), 2.06-2.03 (1H, m), 1.93-1.71 (2H, m), 1.52 (3H, s), 1.17 (3H, s), 1.05 (3H, d, J=7.04 Hz).
13C NMR (CDCl₃), 100 MHz: δ 196.0, 185.7, 172.8, 161.8, 161.7, 157.0, 151.3, 147.1, 143.8, 129.9, 121.0, 120.9, 118.7, 112.0, 100.1, 98.3, 97.2, 87.5, 85.6, 71.5, 71.2, 49.3, 48.2, 48.0, 45.4, 43.2, 36.6, 35.6, 33.9, 33.8, 33.7, 32.9, 32.8, 32.7, 32.6, 23.1, 23.0, 17.2, 16.1, 8.5.
FT-IR values are as follows:
   FT-IR (KBr): 3415, 1729, 1668, 1629, 1608 cm⁻¹.

### Example 3

### Preparation of compound of formula IV-B, wherein the R is furoate

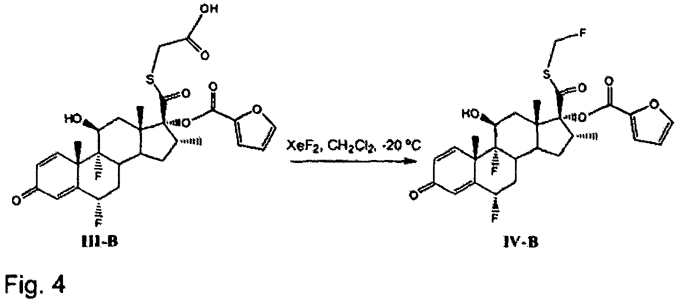

To compound of formula III-B (0.050 g, 0.09 mmol) in dichloromethane (6 mL) at -20 °C XeF₂ (0.030 g, 0.18 mmol) was added and the solution stirred at -20 °C for 2 days. 5% aqueous NaHCO₃ was added (6 mL) and the mixture extracted with dichloromethane (3x6 mL), dried with anhydrous MgSO₄, and concentrated to afford a crude solid containing compound of formula IV-B (0.043 g).

The same procedure can be applied to obtain compound of formula IV-A (fluticasone propionate).

It is evident to one skilled in the art that this invention is not limited to the forgoing examples, and that can be embodied in other specific forms without departing from the scope of the invention. Thus, the examples should be considered as illustrative and not restrictive, reference being made to the claims, and that all changes which come within the meaning and range of equivalency of claims be embraced therein.

## Claims

1. A method of preparing an organic biologically active compound containing a "CH₂F" moiety, which method comprises the steps of: reacting a compound of formula R*-SH with X-acetic acid to yield an intermediate of formula R*-S-CH₂COOH; fluorodecarboxylating the intermediate of formula R*-S-CH₂COOH with a reagent selected from a group consisting of XeF₂ and BrF₃ to yield a compound of formula R*-S-CH₂F,
wherein:
R*SH is an organic multifunctional molecule;
and X is halogen, triflate, mesylate, a fluorosulfonate or a phosphate.

2. A method according to claim 1 wherein R*SH comprises one or more of the following functional groups: ketone, halogen, unsaturated hydrocarbon containing one or more carbon-carbon double bonds, or hydroxyl.

3. A method according to claim 2 wherein the halogen is fluorine.

4. A method according to claim 1, 2 or 3 wherein the compound of formula R*SH is a steroid molecule.

5. A method of preparing an organic biologically active compound according to claim 1, 2, 3 or 4, comprising the steps of: reacting a steroid of formula I with X-acetic acid of formula II to yield an intermediate of formula III; fluorodecarboxylating the intermediate of formula III with a reagent, R1, selected from a group consisting of XeF₂ and BrF₃, to yield compound of formula IV, 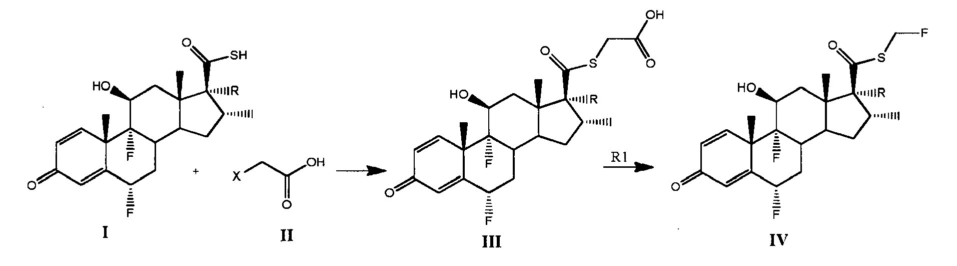 wherein:
R is propionate, furoate or hydroxyl and X is halogen, triflate, mesylate, a fluorosulfonate or a phosphate.

6. A method according to any preceding claim, wherein X is a halogen.

7. A method according to claim 6 wherein the halogen is bromine.

8. A method according to any one of the preceding claims 5 to 7, wherein R is furoate or propionate.

9. A compound of formula III, 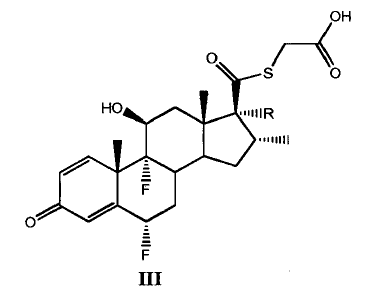 wherein R is propionate, furoate or hydroxyl.

10. A process for preparing an organic biologically active compound having a formula R*S-CH₂F comprising the step of fluorodecarboxylating a compound of formula III according to claim 9, in the presence of a reagent selected from the group consisting of XeF₂ and BrF₃.

11. The process according to claim 10, wherein the organic biologically active compound containing a "CH₂F" moiety is a compound of formula IV, 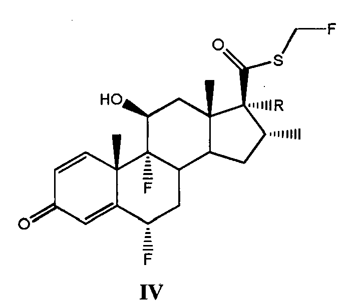 wherein R is furoate or propionate or hydroxyl.

## Patentansprüche

1. Verfahren zur Herstellung einer organischen, biologisch aktiven Verbindung, die einen "CH₂F"-Molekülteil enthält, wobei das Verfahren die folgenden Schritte umfasst: Umsetzen einer Verbindung der Formel R*-SH mit X-Essigsäure, um eine Zwischenverbindung der Formel R*-S-CH₂COOH zu ergeben; Fluordecarboxylieren der Zwischenverbindung der Formel R*-S-CH₂COOH mit einem Reagenz, das aus einer Gruppe bestehend aus XeF₂ und BrF₃ ausgewählt ist, um eine Verbindung der Formel R*-S-CH₂F zu ergeben,
wobei:
R*SH ein organisches multifunktionelles Molekül ist
und X Halogen, Triflat, Mesylat, ein Fluorsulfonat oder ein Phosphat ist.

2. Verfahren nach Anspruch 1, wobei R*SH eine oder mehrere der folgenden funktionellen Gruppen umfasst: Keton, Halogen, ungesättigter Kohlenwasserstoff, der eine oder mehrere Kohlenstoff-Kohlenstoff-Doppelbindungen enthält, oder Hydroxyl.

3. Verfahren nach Anspruch 2, wobei das Halogen Fluor ist.

4. Verfahren nach Anspruch 1, 2 oder 3, wobei die Verbindung der Formel R*SH ein Steroidmolekül ist.

5. Verfahren zur Herstellung einer organischen, biologisch aktiven Verbindung nach Anspruch 1, 2, 3 oder 4, das die folgenden Schritte umfasst:
Umsetzen eines Steroids der Formel I mit X-Essigsäure der Formel II, um eine Zwischenverbindung der Formel III zu ergeben; Fluordecarboxylieren der Zwischenverbindung der Formel III mit einem Reagenz R1, das aus einer Gruppe bestehend aus XeF₂ und BrF₃ ausgewählt ist, um eine Verbindung der Formel IV zu ergeben: 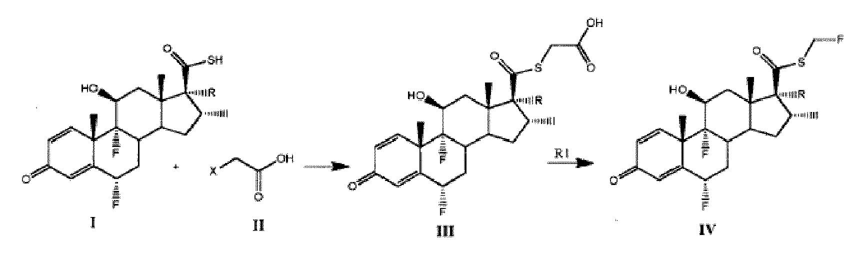 wobei:
R Propionat, Furoat oder Hydroxyl ist und X Halogen, Triflat, Mesylat, ein Fluorsulfonat oder
ein Phosphat ist.

6. Verfahren nach einem vorhergehenden Anspruch, wobei X ein Halogen ist.

7. Verfahren nach Anspruch 6, wobei das Halogen Brom ist.

8. Verfahren nach einem der vorhergehenden Ansprüche 5 bis 7, wobei R Furoat oder Propionat ist.

9. Verbindung der Formel III: 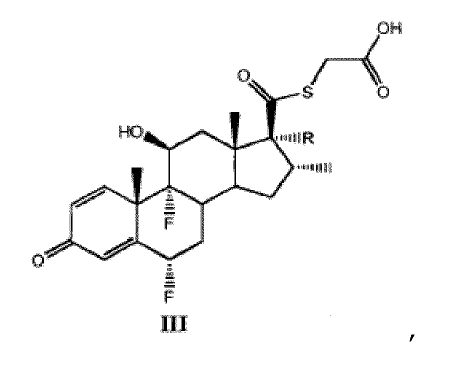 wobei R Propionat, Furoat oder Hydroxyl ist.

10. Verfahren zur Herstellung einer organischen, biologisch aktiven Verbindung mit einer Formel R*S-CH₂F, das den Schritt des Fluordecarboxylierens einer Verbindung der Formel III nach Anspruch 9 in Gegenwart eines Reagenz, das aus der Gruppe bestehend aus XeF₂ und BrF₃ ausgewählt ist, umfasst.

11. Verfahren nach Anspruch 10, wobei die organische, biologisch aktive Verbindung, die einen "CH₂F"-Molekülteil enthält, eine Verbindung der Formel IV ist: 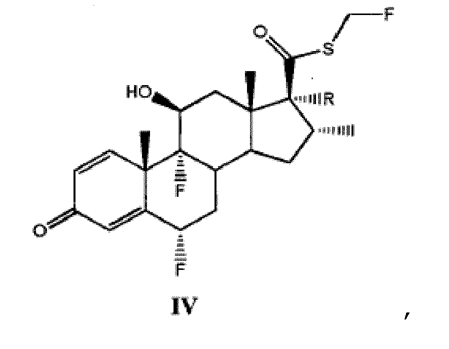 wobei R Furoat oder Propionat oder Hydroxyl ist.

## Revendications

1. Procédé de préparation d'un composé organique biologiquement actif contenant une fraction « CH₂F », lequel procédé comprend les étapes consistant à : faire réagir un composé de formule R*-SH avec de l'acide X-acétique pour produire un intermédiaire de formule R*-S-CH₂COOH ; et fluorodécarboxyler l'intermédiaire de formule R*-S-CH₂COOH avec un réactif choisi dans un groupe constitué par XeF₂ et BrF₃, pour produire un composé de formule R*-S-CH₂F,
dans lequel :
R*SH est une molécule organique multifonctionnelle ; et
X est un halogène, triflate, mésylate, fluorosulfonate ou phosphate.

2. Procédé selon la revendication 1 dans lequel R*SH comprend un ou plusieurs des groupes fonctionnels suivants : cétone, halogène, hydrocarbure insaturé contenant une ou plusieurs doubles liaisons carbone-carbone ou hydroxyle.

3. Procédé selon la revendication 2 dans lequel l'halogène est le fluor.

4. Procédé selon la revendication 1, 2 ou 3 dans lequel le composé de formule R*SH est une molécule de stéroïde.

5. Procédé de préparation d'un composé organique biologiquement actif selon la revendication 1, 2, 3 ou 4, comprenant les étapes consistant à : faire réagir un stéroïde de formule I avec de l'acide X-acétique de formule II pour produire un intermédiaire de formule III ; fluorodécarboxyler l'intermédiaire de formule III avec un réactif, R1, choisi dans un groupe constitué par XeF₂ et BrF₃, pour produire un composé de formule IV, 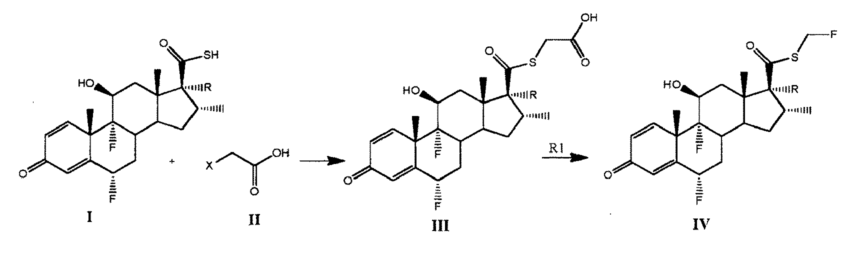 dans lequel :
R est un propionate, furoate ou hydroxyle et X est un halogène, triflate, mésylate, fluorosulfonate ou phosphate.

6. Procédé selon une quelconque revendication précédente, dans lequel X est un halogène.

7. Procédé selon la revendication 6 dans lequel l'halogène est le brome.

8. Procédé selon l'une quelconque des revendications 5 à 7 précédentes, dans lequel R est un furoate ou propionate.

9. Composé de formule III, 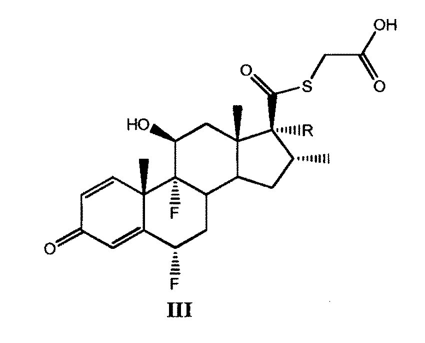 dans lequel R est un propionate, furoate ou hydroxyle.

10. Procédé pour la préparation d'un composé organique biologiquement actif répondant à la formule R*S-CH₂F comprenant l'étape consistant à fluorodécarboxyler un composé de formule III selon la revendication 9, en présence d'un réactif choisi dans le groupe constitué par XeF₂ et BrF₃.

11. Procédé selon la revendication 10, dans lequel le composé organique biologiquement actif contenant une fraction « CH₂F » est un composé de formule IV, 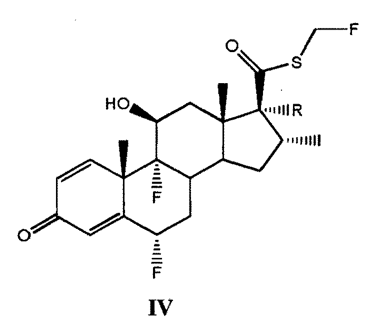 dans lequel R est un furoate ou propionate ou hydroxyle.
